# EUROPEAN PATENT SPECIFICATION

(11) **EP 1 817 428 B1**
(45) Date of publication and mention of the grant of the patent: **08.02.2012**
(21) Application number: 05808595.2
(22) Date of filing: 25.11.2005
(51) Int. Cl.: C12Q 1/68

(54) **A METHOD FOR INCREASING THE SPECIFICITY OF ENZYMATIC DNA SYNTHESIS**
VERFAHREN ZUR ERHÖHUNG DER SPEZIFITÄT ENZYMATISCHER DNA-SYNTHESE
PROCÉDÉ D'AUGMENTATION DE LA SPÉCIFICITÉ DE LA SYNTHÈSE ENZYMATIQUE D'ADN

(30) Priority: 30.11.2004 GB 0426303
(43) Date of publication of application: 15.08.2007
(60) Divisional application: 11173313.5
(73) Proprietor: Bioline Limited, Greater London NW2 6EW (GB)
(72) Inventor: IGNATOV, Konstantin, Moscow, 117246 (RU); KRAMAROV, Vladimir, Moscow, 113303 (RU)
(74) Representative: Kremer, Simon Mark
(86) International application number: PCT/GB2005/004537
(87) International publication number: WO 2006/059075

(56) References cited:
- US-B1- 6 270 962
- STEVENS M ET AL.: "Inhibition of human immunodeficiency virus by a new class of pyridine oxide derivatives" ANTIMICROBIAL AGENTS AND CHEMOTHERAPY, vol. 47, September 2003 (2003-09), pages 2951-2957, XP002369069
- BALZARINI J ET AL: "Pyridine oxide derivatives: Structure-activity relationship for inhibitio of human immunodeficiency virus and cytomegalovirus replication in cell culture" HELVETICA CHIMICA ACTA, VERLAG HELVETICA CHIMICA ACTA. BASEL, CH, vol. 85, no. 9, 2002, pages 2691-2694, XP002247066 ISSN: 0018-019X
- CHEVET E ET AL.: "Low concentrations of tetramethylammonium chloride increase yield and specificity of PCR" NUCLEIC ACIDS RESEARCH, vol. 23, 1995, pages 3343-3344, XP008060132 cited in the application
- HENKE W ET AL.: "Betaine improves the PCR amplification of GC-rich DNA sequences" NUCLEIC ACIDS RESEARCH, vol. 25, 1997, pages 3957-3958, XP002369070 cited in the application

## Description

### Field of the Invention.

The present invention relates to improvements in methods of enzymatic synthesis of nucleic acids by the addition of organic compounds, such as 3-(1-pyridinio)-1-propanesulfonate (PPS), pyridine-N-oxide and others, containing a positive charged N-pyridinium group in molecules. In particular, the invention provides methods and kits for improvements in laboratory procedures using DNA polymerases. The disclosed methods are particularly useful for improving the specificity and yield of polymerase chain reaction (PCR).

### Background to the Invention.

Polymerase chain reaction (PCR) is very important to the development of the biotechnology industry as well as for basic biological research. PCR reactions today are carried out by the use of a heat-resistant DNA polymerase enzyme (such as Taq DNA polymerase) in a multi-cycle process employing several alternating heating and cooling steps to amplify DNA (U.S. Pat. Nos. 4,683,202 and 4,683,195). First, a reaction mixture is heated to a temperature sufficient to denature the double stranded target DNA into its two single strands. The temperature of the reaction mixture is then decreased to allow specific oligonucleotide primers to anneal to their respective complementary single-stranded target DNA. Following the annealing step, the temperature is raised to the temperature optimum of the DNA polymerase being used, which allows incorporation of complementary nucleotides at the 3' ends of the annealed oligonucleotide primers thereby recreating double stranded target DNA. Using a heat-stable DNA polymerase, the cycle of denaturing, annealing and extension may be repeated as many times as necessary to generate a desired product, without the addition of polymerase after each heat denaturation. Twenty or thirty replication cycles can yield up to a million-fold amplification of the target DNA sequence ("Current Protocols in Molecular Biology," F.M. Ausubel et al. (Eds.), John Wiley and Sons, Inc., 1998).

Although PCR technology has had a profound impact on biomedical research and genetic identity analysis, amplification of non-target oligonucleotides and mispriming on non-target background DNA, RNA, and/or the primers themselves, still present significant problems. This is especially true in diagnostic applications where PCR is carried out in a milieu of complex genetic backgrounds where the target DNA may be proportionately present at a very low level (Chou et al., Nucleic Acid Res., 20:1717-1723 (1992), and in PCR-amplifications of GC-rich DNA templates. A chief problem is that primers may prime extensions at non-specific sequences because only a few base pairs at the 3'-end of a primer, which are complementary to a DNA sequence, can result in a stable priming complex. As a result, competitive or inhibitory products can be produced at the expense of the desired product.

The numerous protocols designed for different PCR applications almost always require an optimization step for implementation (Dieffenbach, C.W. and Dveksler,G.S. (1995) PCR Primer: A Laboratory Manual. Cold Spring Harbor Laboratory Press, Cold Spring Harbor, New York, NY.). In some cases, especially when complex cDNA or genomic DNA is used as the PCR template and the calculated annealing temperature is relatively low, multiple non-specific bands cannot be eliminated although all experimental parameters are seemingly optimized. It has been reported that some reagents like tetramethylammonium (TMA) chloride (Chevet, E., et al, (1995) Nucleic Acids Res., 23, 3343-3344; Hung, T., et al, (1990) Nucleic Acids Res., 18, 4953; Warner, C.K. and Dawson, J.E. (1996) In Persing, D.H. (ed.), PCR Protocols for Emerging Infectious Diseases. ASM Press, Washington DC.), dimethyl sulfoxide (Winship, P.R. (1989) Nucleic Acids Res., 17, 1266; Bookstein, R., et al, (1990) Nucleic Acids Res., 18, 1666; Sidhu, M.K., et al, (1996) Biotechniques, 21, 44-47.), amides (Chakrabarti, R., et al, (2001) Nucleic Acids Res., 29, 2377-2381) and betaine (Weissensteiner, T., et al, (1996) Biotechniques, 21, 1102-1108; Henke, W., et al, (1997) Nucleic Acids Res., 19, 3957; U.S. Pat. Nos. 6,270,962) are capable of improving the efficacy and specificity of PCR. These reagents are often used as components of the commercial optimization and enhancer kits for PCR.

Unfortunately, in a number of cases, the above mentioned compounds are unable to suppress non-specific PCR products or increase the yield of specific fragments. Thus, there is a need for new compounds for use as PCR enhancers.

### Disclosure of the Invention.

The present invention provides a method for improvement of DNA enzymatic synthesis. In particular, the invention relates primarily to the improvement of PCR specificity and yield by the addition of organic compounds containing a positive charged pyridinium group in their molecules, , pyridinium inner salts (e.g. 3-(1-pyridinio)-1-propanesulfonate), pyridine-N-oxide and others. The invention also can be useful for improving other laboratory procedures using DNA polymerases, such as primer extension and DNA sequencing.

Although specific pyridine N-oxide derivatives have been used as additives to inhibit the enzymatic activity of defined polymerase reactions, i.e. HIV reverse transcriptase, such pyridinium compounds have not been reported as components within polymerase reactions to improve specificity and yield (Stevens, M. et al., (2003) Antimicrobial Agents Chemother., 47, 2952-2957).

The present invention provides methods, regents and reaction kits for suppressing the non-specific products and/or increasing the yield of specific products in reactions catalyzed by DNA polymerase. The decrease in non-specific products and the increase in specific products of the reactions are achieved by the addition of organic compounds containing a positive charged N-pyridinium group in molecules, which are described herein, to the reaction mixtures.

In particular, the invention provides a method of improving a DNA polymerase reaction by including in a reaction mixture containing a DNA polymerase pyridine N-oxide or 3-(1-pyridino)-1-propansulfonate.

The present invention allows improving the efficacy and specificity of DNA polymerase reactions, such as primer extension reaction, reverse-transcription reaction, DNA sequencing, nick-translation, PCR and other reactions which are catalyzed by DNA polymerases.

The organic compounds containing a positive charged N-pyridinium group in molecules, which are capable of improving the efficacy and specificity of DNA polymerase reactions, may be pyridinium inner salts (e.g. 3-(1-pyridinio)-1-propanesulfonate), pyridine-N-oxide and others described herein.

The DNA polymerases, which are used for performing the DNA polymerase reactions, can be RNA-dependent DNA polymerases, such as AMV or MMLV reverse-transcriptase, and DNA-dependent DNA polymerases, such as Klenow fragment, DNA Polymerase I *E.coli,* Taq, Tth or Pfu DNA polymerase.

The reaction kits of the invention contain the organic compounds, which are necessary to improve the specificity and/or efficacy of DNA polymerase reactions and described herein. Furthermore, the reaction kit may contain a plurality of additional reaction components. Among the additional reaction components, one may include an enzyme, such as a DNA polymerase for PCR, reverse-transcription or DNA sequencing.

Other features, aspects and advantages of the invention will be, or will become, apparent to one with skill in the art upon examination of the following figures and detailed description. It is intended that all such additional systems, features, aspects and advantages included within this description, are within the scope of the invention, and are protected by the following claims.

### Brief Description of the Figures.

These and other features, aspects, and advantages of the present invention will become better understood with regard to the following description, claims and accompanying drawings where:

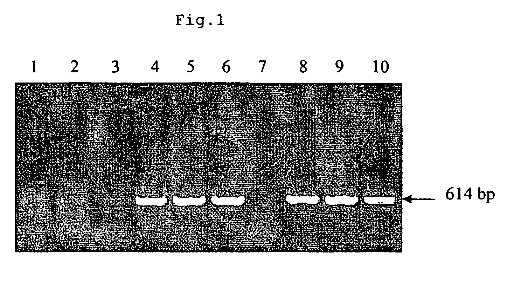

FIG. 1 depicts an electrophoretic analysis of the PCR products obtained in Example 1. A 614-bp DNA fragment was amplified from 25 ng of *Gallus domesticus* genomic DNA for 30 cycles. PCR was performed under conventional conditions without extra additives (lane 1), in presence of 0.1 M, 0.2 M, 0.3 M, 0.4 M, 0.5 M pyridine-N-oxide (lanes 2, 3, 4, 5, 6), and in presence of 0.1 M, 0.2 M, 0.3 M, 0.4 M 3-(1-pyridinio)-1-propanesulfonate (lanes 7, 8, 9, 10).

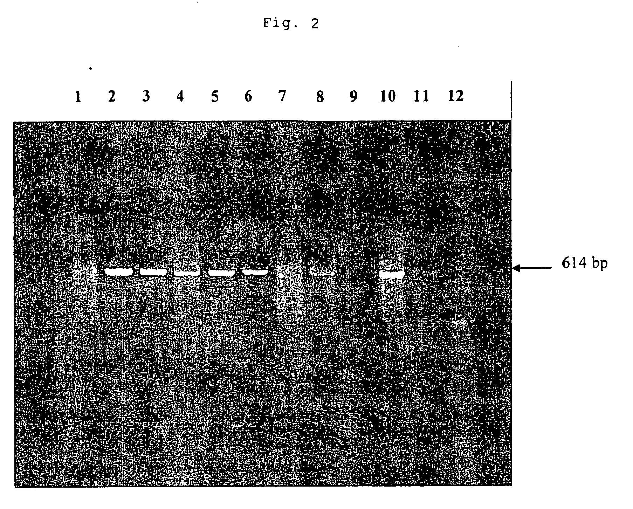

FIG. 2 depicts an electrophoretic analysis of the PCR products obtained in Example 2. A 614-bp DNA fragment was amplified from 25 ng of *Gallus domesticus* genomic DNA for 30 cycles. PCR was performed under conventional conditions without extra additives (lane 1); in presence of 0.2 M and 0.4 M 3-(1-pyridinio)-1-propanesulfonate (lanes 2 and 3); in presence of 0.3 M, 0.6 M and 1.2 M betaine (lanes 4, 5, and 6); in presence of 12.5 mM, 25 mM and 50mM tetramethylammonium chloride (TMA-Cl) (lanes 7, 8, and 9); and in presence of 0.7 M, 1.4 M and 2.1 M DMSO (lanes 10, 11, and 12).

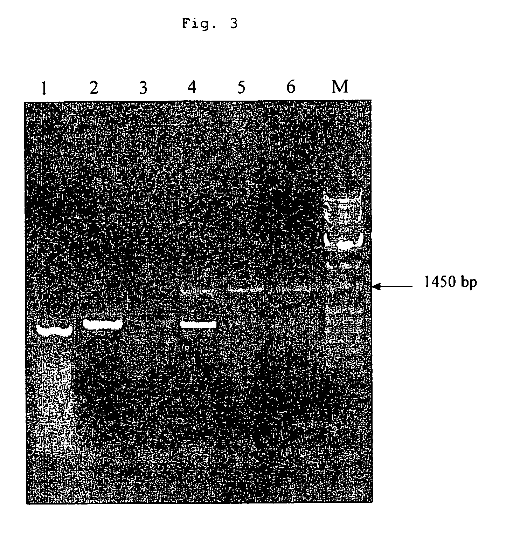

FIG. 3 depicts an electrophoretic analysis of the PCR products obtained in Example 3. A 1450-bp DNA fragment of *X. laevis* cDNA was amplified from 100 ng of Xenopus *laevis* embryo MATCHMAKER LexA cDNA library (Clontech Laboratories) in 40 cycles. PCR was performed under conventional conditions without extra additives (lane 1); in presence of 1.25 M and 1.7M betaine (lanes 2 and 3); and in presence of 0.3 M, 0.35 M and 0.4 M 3-(1-pyridinio)-1-propanesulfonate (lanes 4, 5 and 6). Lane M - DNA marker.

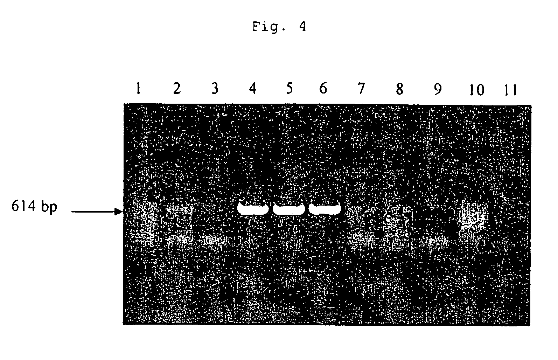

FIG. 4 depicts an electrophoretic analysis of the PCR products obtained in Example 4. A 614-bp DNA fragment was amplified from 25 ng of *Gallus domesticus* genomic DNA for 30 cycles. PCR was performed under conventional conditions without extra additives (lane 1); in presence of 0.1 M, 0.2 M, 0.3 M, 0.4 M and 0.5M pyridine-N-oxide (lanes 2, 3, 4, 5 and 6); and in presence of 0.1 M, 0.2 M, 0.3 M, 0.4 M and 0.5M trimethylamine-N-oxide (lanes 7, 8, 9, 10 and 11).

### Detailed Description of the Invention.

In order to provide a clear and consistent understanding of the specification and claims, the following definitions are provided.

The term "nucleic acids", as used herein, refers to either DNA or RNA. It includes plasmids, chromosomal DNA or RNA, nonfunctional DNA or RNA, and DNA or RNA synthesized in vitro.

The term "nucleic acid sequence" or "polynucleotide sequence" refers to a single- or double-stranded polymer of deoxyribonucleotide or ribonucleotide bases read from the 5' to the 3' end.

The terms "oligonucleotide primer", "oligonucleotide" or "primer" refer to a single-stranded polymer of deoxyribonucleotides or ribonucleotides.

The term "complementary" as used herein refers to a relationship between two nucleic acid sequences. One nucleic acid sequence is complementary to a second nucleic acid sequence if it is capable of forming a duplex with the second nucleic acid, wherein each residue of the duplex forms a guanosine-cytidine (G-C) or adenosine-thymidine (A-T) base pair or an equivalent base pair. Equivalent base pairs can include nucleoside or nucleotide analogues other than guanosine, cytidine, adenosine, or thymidine.

Terms "thermostable", "thermally stable" and "heat-stable" are used interchangeably herein to describe enzymes, which can withstand temperatures up to at least 95°C for several minutes without becoming irreversibly denatured. Typically, such enzymes have an optimum temperature above 45°C, preferably between 50° to 75°C.

The term "modification" of an enzyme as used herein, refers to a chemical or genetic modification of enzyme.

The terms "DNA template", "RNA template" or "template" as used herein, refer to a nucleic acid that is used by a polymerase to synthesize a new complementary nucleic acid.

The term "betaine", as used herein, refers to N,N,N-trimethylglycine.

The term "pyridinium betain", as used herein, refers to inner salts of pyridinium.

Some compounds of the invention may be present with a positive or negative charge or with both a positive and negative charges, depending on the pH of the solution. It is understood that these various forms of these compounds are included in the present invention.

In one aspect, the present invention provides reagents and methods for suppressing the non-specific products and increasing the yield of specific products in DNA polymerase reactions. These reactions can be a primer extension reaction, a reverse-transcription reaction, DNA sequencing, nick-translation, PCR and other reactions, which are catalyzed by DNA polymerases. Suppressing the non-specific products and increasing the yield of specific products of the reactions are achieved by the addition of organic compounds containing a positive charged pyridinium group. These compounds, which are capable of improving the efficacy and specificity of DNA polymerase reactions, can be pyridinium inner salts (*e.g*. 3-(1-pyridinio)-1-propanesulfonate), pyridine-N-oxide and others.

In one aspect, the present invention provides a reaction kit for increasing the specificity and/or efficacy of DNA polymerase reactions. The kit may include an organic compound (or compounds) containing a positive charged pyridinium group in its molecules, or a complex reagent containing this compound, or solution of the compound or the reagent. The kit may further include one or more additional reaction components to facilitate the enzymatic process. The kit may further include one or more DNA polymerases for performing the enzymatic process.

Generally, a kit may comprise a first container containing a compound of the formula I and at least a second container having one or more components suitable for performing a DNA polymerase reaction, including a DNA polymerase enzyme. The second container may contain one of more of (a) dNTPs; (b) ddNTPs; (c) reaction buffer(s) and (d) a primer. The kit may contain two or more, e.g. three, four or five separate containers with these or other components packaged separately or in combinations thereof. Kits may also contain instructions for use of the reagents.

The kit will usually contain the compound of formula I in a concentrated solution, e.g. at about 5x to 10x the reaction concentration.

Suitable DNA polymerases for use in the methods, kits and other aspects of the invention include E. coli DNA Polymerase I, Klenow enzyme, T4 DNA Polymerase, and T7 DNA Polymerase. These polymerases may be of wild-type sequences or synthetic variants and fragments.

The polymerase may be a reverse transcriptase, such as AMV or MMLV reverse-transcriptase, including synthetic variants or fragments thereof.

In a preferred aspect, the DNA polymerase will be a thermally stable DNA polymerase. This includes, but is not limited to, *Thermus aquaticus* DNA polymerase; N-terminal deletions of *Taq* polymerase, including the Stoffel fragment of DNA polymerase, Klentaq-235, and Klentaq-278; *Thermus thermophilus* DNA polymerase; *Bacillus caldotenax* DNA polymerase; *Thermus flavus* DNA polymerase; *Bacillus stearothermophilus* DNA polymerase; and archaebacterial DNA polymerases, such as *Thermococcus litoralis* DNA polymerase (also referred to as Vent_{R}^{®}), Pfu, Pfx, Pwo, and DeepVent_{R}^{®} or a mixture thereof. Other commercially available polymerases DNA polymerases include TaqLA or Expand High Fidelity^{Plus} Enzyme Blend (Roche); KlenTaqLA, KlenTaq1, TthLA (Perkin-Elmer), ExTaq® (Takara Shuzo); Elongase® (Life Technologies); Taquenase™ (Amersham), TthXL (Perkin Elmer); Advantage™ KlenTaq and Advantage™ Tth (Clontech); TaqPlus® and TaqExtender™ (Stratagene); or mixtures thereof.

Methods for using compounds containing a positive charged pyridinium group for increasing the specificity and/or efficacy of DNA polymerase reactions are also provided in which the compound is added to a reaction mixture. Additional steps include incorporation of additional reaction components, which may include template nucleic acid(s), oligonucleotide primer(s), polymerase(s) and others, and adjusting the reaction conditions (e.g., increased temperature) to activate and/or complete an enzymatic process.

In a preferred embodiment, the present invention includes reagents and methods for increasing the specificity of PCR. Specifically, the present invention provides processes and kits for performing a specific PCR. The processes and kits utilize the step of addition of the organic compounds, which are capable of improving the PCR specificity and described herein, to the reaction mixture of PCR.

PCR is a polymerase chain reaction described in US Patents Nos. 4,683,202 and 4,683,195. The PCR employs a heating and cooling cycle to drive the reaction. First, the reaction mixture is heated to, or above, the nucleic acid melting temperature, then cooled to allow specific oligonucleotide primers to bind to the sample (annealing), and then heated to optimize the addition of complementary bases to the amplified nucleic acid (extension). The use of a heat-stable DNA polymerase (U.S. Pat. No. 4,889,818) allows to repeat this cycle of denaturing, annealing and extension as many times as needed to generate the desired product. Twenty replication cycles can yield up to a million-fold amplification of the target DNA sequence.

Preferred DNA polymerases for use in PCR applications include thermally stable DNA polymerases and/or combinations thereof. Thermally stable DNA polymerases may include, but are not limited those mentioned herein above.

The addition of an appropriate amount of one or more of the compounds described herein facilitates the improving the efficacy and specificity of PCR. See examples herein, for a demonstration of the effects of 3-(1-pyridinio)-1-propanesulfonate and pyridine-N-oxide on PCR efficacy and specificity. Other compounds of the invention can be used in a similar manner to improve efficacy and specificity of PCR methods

As indicated above, the compounds used in the present invention are N-pyridinium group containing organic molecules or ions that are capable of eliminating or reducing the non-specific products of PCR and other laboratory procedures using DNA polymerases, such as reverse-transcription or DNA sequencing. These compounds are represented by e.g. pyridine N-oxide or 3-(1-pyridinio)-1-propanesulfonate.

Some compounds, may be present with a positive or with both a positive and negative charges, depending on the pH of the solution. It is understood that these various forms of these compounds are included in the present invention.

Preferred compounds for improving PCR applications can be selected from, the group compounds set out in table 1:

| Compound | R1 | R2 | R3 | R4 | R5 | R6 |
|---|---|---|---|---|---|---|
| 3-(1-pyridino)-1-propanesulfonate; | H | H | H | H | H | -(CH₂)₃-SO₃H |
| pyridine-N-oxide | H | H | H | H | H | -O⁻ |

In general, the compounds used to improve PCR, DNA sequencing and other laboratory procedures using a DNA polymerase are commercially available. For example, 3-(1-pyridino)-1-propanesulfonat, pyridine-N-oxide, can all be obtained from Fluka, These compounds may also be synthesized by routine methods known to those of skill in the art.

In a preferred embodiment, the present invention includes methods for increasing the specificity of PCR. Specifically, the present invention provides processes, reagents and kits for suppressing the non-specific products and/or increasing the yield of specific PCR products. The processes, reagents and kits utilize the organic compounds containing a positive charged N-pyridinium group in molecules. By using methods of the present invention, the PCR-amplification specificity of the target DNA molecules is increased.

The present procedures and reagents can be applied to PCR processes as set forth in Examples.

The principles, methodologies and examples described herein (and below) for performing PCR may be applied in an analogous fashion to perform various types of DNA polymerase reactions described above.

To perform a DNA polymerase reaction according to the present invention, a reaction mixture which includes a DNA polymerase is provided, together with a suitable concentration of a compound of formula I. Typically, the compound of formula I (or mixture of such compounds) may be provided at a final concentration of from 0.05 to 1.0 M, preferably from 0.1 to 0.9 M, such as from 0.2 to 0.75 M, more preferably from 0.2 to 0.5 M, e.g. from 0.25 to 0.5 M.

In addition to the DNA polymerase the reaction mixture typically contains other components conventional in the art such as a DNA template, one or more primers, dNTPs and a buffer for the polymerase. These may be provided by a person of skill in the art using routine skill and knowledge and the precise nature and amounts of these components is not essential as such to the present invention.

The following Examples illustrate aspects of the invention. The Examples are illustrative of, but not binding on, the present invention. Any methods, preparations, solutions and such like, which are not specifically defined, may be found in Sambrook et al. All solutions are aqueous and made up in sterile, deionised water, unless otherwise specified. All enzymes were obtained from Promega Corporation.

### EXAMPLES

### Example 1

The improvement of PCR specificity and yield by adding compounds containing a positively charged pyridinium group in molecules (pyridine-N-oxide and 3-(1-pyridinio)-1-propanesulfonate).

A 614-bp DNA fragment was amplified from 25 ng of *Gallus domesticus* genomic DNA in 30 cycles: 95°C - 30 sec; 58°C - 30 sec; 72°C - 30 sec. The reaction mixture (50 µl) contained: 1.5 mM MgCl₂, 20 mM Tris-HCl (pH 9.0 at 25°C.), 50 mM KCl, 0.1% Triton X-100, 0.2 mM each dNTP, 20 pmol primer Pr1 (5'-attactcgagatcctggacaccagc), 20 pmol primer Pr2 (5'-attaggatcctgccctctcccca), and 2.5U Taq DNA polymerase.

PCR reactions were performed in the absence of any extra additives and in the presence of extra additives: pyridine-N-oxide or 3-(1-pyridinio)-1-propanesulfonate. The extra additives were added to the reaction mixture up to final concentrations: 0.1 M, 0.2 M, 0.3 M, 0.4 M, 0.5 M pyridine-N-oxide, and 0.1 M, 0.2 M, 0.3 M, 0.4 M 3-(1-pyridinio)-1-propanesulfonate.

**FIG. 1** depicts the electrophoretic analysis of the amplification products obtained. Under these reaction conditions only the method of present invention (addition of the compounds containing a positive charged pyridinium group in molecules) provided a detectable amount of the desired product (increased yield of PCR) and elimination of non-specific amplification products (increased specificity of PCR). Compared to the conventional PCR procedure without extra additives (lane 1), target DNA synthesis and elimination of non-specific PCR products were obtained by using pyridine-N-oxide at concentrations from 0.3 M to 0.5 M (lanes 4-6) or 3-(1-pyridinio)-1-propanesulfonate at concentrations from 0.2 M to 0.4 M (lanes 8-10) (note the presence of the target amplification product and the absence of non-specific products in lanes 4-6 and lanes 8-10 compared to lane 1).

### Example 2

Comparing the ability of betaine, tetramethylammonium chloride (TMA-C1), dimethyl sulfoxide (DMSO) and 3-(1-pyridinio)-1-propanesulfonate to improve the specificity of PCR.

Betaine, tetramethylammonium chloride (TMA-C1) and dimethyl sulfoxide (DMSO) are the most popular additives to DNA polymerase reaction mixtures (e.g. PCR and DNA Sequencing mixtures), which are used for improving specificity and yield of the reactions. The ability of betaine, TMA-C1, DMSO and claimed in this invention 3-(1-pyridinio)-1-propanesulfonate to improve specificity and yield of a DNA polymerase reaction were compared in PCR-amplification of 614-bp DNA fragment from *Gallus domesticus* genomic DNA.

PCR was performed as described in the Example 1. The reaction mixtures contained or did not contain the extra additives.

**FIG. 2** depicts the electrophoretic analysis of the amplification products obtained. Under these reaction conditions only the presence of 0.2 - 0.4 M 3-(1-pyridinio)-1-propanesulfonate (lanes 2 and 3) and betaine at concentrations higher than 1 M (note lane 6 - 1.2 M betaine) provided an elimination of non-specific amplification products along with a detectable amount of the desired product.

It should be noted that 0.2 M 3-(1-pyridinio)-1-propanesulfonate provides the same effect as 1.2 M betaine. An additional comparison between betaine and 3-(1-pyridinio)-1-propanesulfonate is described in Example 3.

### Example 3

Comparing the ability of betaine (claimed in U.S. Pat. Nos. 6,270,962) and 3-(1-pyridinio)-1-propanesulfonate (claimed in this invention) to improve PCR-amplification of DNA.

A 1450-bp DNA fragment of *X. laevis* cDNA was amplified from 100 ng of *Xenopus laevis* embryo MATCHMAKER LexA cDNA library (Clontech Laboratories) in 40 cycles: 94°C - 30 sec; 58°C - 35 sec; 72°C - 60 sec. The reaction mixture (50 µl) contained: 1.5 mM MgCl₂, 20 mM Tris-HCl (pH 9.0 at 25°C.), 50 mM KCl, 0.1% Triton X-100, 0.2 mM each dNTP, 20 pmol primer Pr3 (5'-attaccatggacccagcggctcctgccacc), 20 pmol primer Pr4 (5'-ccgctcgagagctcctctacctccttc), and 2.5U Taq DNA polymerase.

PCR reactions were performed in the absence of any extra additives and in the presence of the following extra additives: 1.25 M or 1.7 M betaine, or 0.3 M, 0.35 M or 0.4 M 3-(1-pyridinio)-1-propanesulfonate.

**FIG. 3** depicts the electrophoretic analysis of the amplification products obtained. Under these reaction conditions only the presence of 3-(1-pyridinio)-1-propanesulfonate (lanes 4-6) provided a detectable amount of the desired product. Compared to the conventional PCR procedure without extra additives (lane 1), the additions of both betaine (lanes 2 and 3) and 3-(1-pyridinio)-1-propanesulfonate (lanes 4-6) to the PCR mixture provided a decrease of non-specific amplification products, however, complete elimination of non-specific products was achieved only by 0.4 M 3-(1-pyridinio)-1-propanesulfonate (lane 6).

### Example 4

Comparing the ability of trimethylamine-N-oxide (claimed in U.S. Pat. Nos. 6,270,962) and pyridine-N-oxide (claimed in this invention) to increase PCR specificity and yield.

Two N-oxide compounds, trimethylamine-N-oxide and pyridine-N-oxide, were tested as compounds increasing specificity and yield of a DNA polymerase reaction, such as PCR. Trimethylamine-N-oxide was described in U.S. Pat. Nos. 6,270,962 as a compound improving a DNA polymerase reaction, such as DNA sequencing. Claimed herein pyridine-N-oxide contains a positive charged pyridinium group instead of a positive charged N,N,N-trimethyl group in trimethylamine-N-oxide.

The PCR was performed as described in the Example 1. The reaction mixtures contained from 0.1 M to 0.5 M pyridine-N-oxide, or from 0.1 M to 0.5 M trimethylamine-N-oxide, or did not contain the extra additives.

**FIG. 4** depicts the electrophoretic analysis of the amplification products obtained. Under these reaction conditions only the presence of 0.3 - 0.5 M pyridine-N-oxide (lanes 4-6) provided elimination of non-specific amplification products along with obtaining a detectable amount of the desired product. Compared to the conventional PCR procedure without extra additives (lane 1), trimethylamine-N-oxide at concentrations from 0.1 M to 0.5 M (lanes 7-11) did not provide a considerable improvement of PCR.

It is to be understood that the above-described methods are merely representative embodiments illustrating the principles of this invention and that other variations in the methods may be devised by those skilled in the art without departing from the spirit and scope of this invention.

## Claims

1. A method of improving a DNA polymerase reaction by including in a reaction mixture containing a DNA polymerase pyridine-N-oxide or 3-(1-pyridinio)-1-propanesulfonate, wherein said DNA polymerase reaction is a PCR.

2. The method according to claim 1 wherein the reaction mixture comprises a DNA molecule having trinucleotide repeats where the trinucleotides are GC rich.

3. A kit for improving a DNA polymerase reaction comprising in separate containers:
a) components for DNA polymerase reaction, including a DNA polymerase enzyme
b) a container which contains one or more of pyridine-N-oxide or 3-(1-pyridinio)-1-propanesulfonate.

4. The kit of claim 3 wherein said DNA polymerase enzyme is:
(a) selected from the group consisting of *E. coli* DNA Polymerase I, Klenow enzyme, T4 DNA Polymerase, and T7 DNA Polymerase;
(b) a reverse transcriptase; or
(c) a thermostable DNA polymerase.

5. The kit of claim 4 part (b) wherein said reverse transcriptase is selected from the group consisting of AMV and M-MuLV.

6. The kit of claim 4 part (c) wherein said thermostable DNA polymerase is selected from the group consisting of Taq DNA polymerase; N-terminal deletions of Taq polymerase, Tth DNA polymerase, *Bacillus caldotenax* DNA polymerase; *Thermus flavus* DNA polymerase; *Bacillus stearothermophilus* DNA polymerase; and archaebacterial DNA polymerases, such as Pfu DNA polymerase, Pfx DNA polymerase, Pwo DNA polymerase, *Thermococcus litoralis* DNA polymerase, and DeepVent_{R}^{®}.

7. In a method of performing a DNA polymerase reaction, the improvement comprising including in a reaction mixture containing a DNA polymerase pyridine-N-oxide or 3-(1-pyridinio)-1-propanesulfonate.

## Patentansprüche

1. Verfahren zur Verbesserung einer DNA-Polymerasereaktion durch Miteinbeziehen von Pyridin-N-oxid oder 3-(1-Pyridinio)-1-propansulfonat in ein Reaktionsgemisch, das eine DNA-Polymerase enthält, worin die DNA-Polymerasereaktion eine PCR ist.

2. Verfahren nach Anspruch 1, worin das Reaktionsgemisch ein DNA-Molekül mit Trinucleotidwiederholungen umfasst, wobei die Trinucleotide GC-reich sind.

3. Set zur Verbesserung einer DNA-Polymerasereaktion, das in getrennten Behältern Folgendes umfasst:
a) Komponenten für eine DNA-Polymerasereaktion, einschließlich eines DNA-Polymeraseenzyms,
b) einen Behälter, der eines oder mehrere von Pyridin-N-oxid und 3-(1-Pyridinio)-1-propansulfonat enthält.

4. Set nach Anspruch 3, worin das DNA-Polymeraseenzym:
(a) aus der aus *E.-coli*-DNA-Polymerase I, Klenow-Enzym, T4-DNA-Polymerase und T7-DNA-Polymerase bestehenden Gruppe ausgewählt ist,
(b) eine reverse Transkriptase ist oder
(c) eine thermostabile DNA-Polymerase ist.

5. Set nach Anspruch 4, Abschnitt (b), worin die reverse Transkriptase aus der aus AMV und M-MuLV bestehenden Gruppe ausgewählt ist.

6. Set nach Anspruch 4, Abschnitt (c), worin die thermostabile DNA-Polymerase aus der aus Taq-DNA-Polymerase, N-terminalen Deletionen von Taq-Polymerase, Tth-DNA-Polymerase, *Bacillus-caldotenax*-DNA-Polymerase, *Thermus-flavus-DNA-*Polymerase, *Bacillus-stearothermophilus*-DNA-Polymerase und Archaebakterien-DNA-Polymerasen, wie z.B. Pfu-DNA-Polymerase, Pfx-DNA-Polymerase, Pwo-DNA-Polymerase, *Thermococcus-litoralis*-DNA-Polymerase und DeepVent_{R}^{®}, bestehenden Gruppe ausgewählt ist.

7. Verbesserung eines Verfahrens zur Durchführung einer DNA-Polymerasereaktion, die das Miteinbeziehen von Pyridin-N-oxid oder 3-(1-Pyridinio)-1-propan-sulfonat in ein Reaktionsgemisch, das eine DNA-Polymerase enthält, umfasst.

## Revendications

1. Procédé pour améliorer une réaction par ADN polymérase par incorporation, dans un mélange réactionnel contenant une ADN polymérase, de pyridine-N-oxyde ou de 3-(1-pyridino)-1-propanesulfonate, dans lequel ladite réaction par ADN polymérase est une PCR.

2. Procédé selon la revendication 1, dans lequel le mélange réactionnel comprend une molécule d'ADN ayant des répétitions de trinucléotides, où les trinucléotides sont riches en GC.

3. Kit pour améliorer une réaction par ADN polymérase comprenant, dans des récipients séparés :
a) des composants pour la réaction par ADN polymérase, comprenant une enzyme ADN polymérase,
b) un récipient qui contient un ou plusieurs parmi un pyridine-N-oxyde et un 3-(1-pyridino)-1-propane-sulfonate.

4. Kit selon la revendication 3, dans lequel ladite enzyme ADN polymérase est :
(a) choisie dans le groupe constitué par l'ADN polymérase I d'*E. coli*, l'enzyme de Klenow, l'ADN polymérase T4, et l'ADN polymérase T7 ;
(b) une transcriptase inverse ; ou
(c) une ADN polymérase thermostable.

5. Kit selon la revendication 4, partie (b), dans lequel ladite transcriptase inverse est choisie dans le groupe constitué par AMV et M-MuLV.

6. Kit selon la revendication 4, partie (c), dans lequel ladite ADN polymérase thermostable est choisie dans le groupe constitué par l'ADN polymérase Taq ; les délétions N-terminales de polymérase Taq ; l'ADN polymérase Tth, l'ADN polymérase de *Bacillus caldotenax ;* l'ADN polymérase de *Thermus flavus* ; l'ADN polymérase de *Bacillus stearothermophilus* ; et les ADN polymérases d'archaebactéries, telles que l'ADN polymérase Pfu, l'ADN polymérase Pfx, l'ADN polymérase Pwo, l'ADN polymérase de *Thermococcus litoralis,* et Deepvent_{R}^{®}.

7. Dans un procédé pour effectuer une réaction par ADN polymérase, l'amélioration comprenant l'incorporation, dans un mélange réactionnel contenant une ADN polymérase, de pyridine-N-oxyde ou de 3-(1-pyridino)-1-propane-sulfonate.
